Europäisches Patentamt

European Patent Office

Office européen des brevets

(19)

(11) Numéro de publication: **0 162 739**
**B1**

## FASCICULE DE BREVET EUROPEEN

(12)

(45) Date de publication du fascicule du brevet:
**01.04.87**

(21) Numéro de dépôt: **85400710.1**

(22) Date de dépôt: **10.04.85**

(51) Int. Cl.⁴: **C 13 F 1/02,** C 13 G 1/06,
B 01 D 9/00

(54) Procédé de conduite automatisée d'un appareil de cristallisation à marche continue pour la production de sucre.

(30) Priorité: **11.04.84 FR 8405743**

(43) Date de publication de la demande:
**27.11.85 Bulletin 85/48**

(45) Mention de la délivrance du brevet:
**01.04.87 Bulletin 87/14**

(84) Etats contractants désignés:
**AT BE DE GB IT NL**

(56) Documents cités:
**AU - B - 520 244**
**FR - A - 2 101 257**
**US - A - 4 155 774**
**US - A - 4 263 010**

(73) Titulaire: **FIVES-CAIL BABCOCK, Société anonyme, 7 rue Montalivet, F-75383 Paris Cedex 08 (FR)**

(72) Inventeur: **Chielens, Alain, 37, rue de la Briqueterie, F-59420 Mouvaux (FR)**
Inventeur: **Journet, Gérard, 9, rue de l'Houssoye, F-59310 Coutiches (FR)**

(74) Mandataire: **Fontanié, Etienne, FIVES-CAIL BABCOCK 7, rue Montalivet, F-75383 Paris Cedex 08 (FR)**

ACTORUM AG

## Description

La présente invention concerne les appareils de cristallisation par évaporation à marche continue constitués par une cuve fermée disposée horizontalement et divisée par des cloisons verticales en plusieurs compartiments munis d'éléments, tels que des plaques creuses ou des tubes, chauffés par de la vapeur, et qui sont traversés successivement par le mélange de cristaux et de liqueur mère. Une solution sucrée sous-saturée, appelée liqueur standard ou égout, est introduite dans chacun des compartiments et un magma formé d'un mélange de petits cristaux et de liqueur mère est introduit dans le premier compartiment; la masse cuite produite est extraite du dernier compartiment.

Pour automatiser la marche de ces appareils, on a prévu de régler l'alimentation en solution sous-saturée ou la pression de la vapeur de chauffage de chaque compartiment ou groupe de compartiments de façon à obtenir une masse cuite ayant la composition voulue; on a également proposé de contrôler la sursaturation de la liqueur mère dans un ou plusieurs compartiments de cristallisation (voir, par exemple: AU-B-520 244). Mais, compte tenu des temps de séjour très longs et de la mauvaise représentativité des paramètres mesurés, les systèmes de régulation classiques ne permettent pas une conduite fiable en régime transitoire.

Le but de la présente invention est d'optimiser la conduite de l'appareil de cristallisation en prenant en compte, pour la détermination des valeurs de consigne des grandeurs réglées, les caractéristiques de la solution sucrée et du magma alimentant l'appareil de cristallisation et les caractéristiques désirées pour la masse cuite produite, ainsi que les paramètres caractérisant le fonctionnement de l'appareil (pression de calandre, débit de vapeur d'agitation, niveau de masse cuite, etc.). Cette optimisation permet d'augmenter la production pour une qualité donnée de sucre, de réduire les pertes dans les mélasses et de diminuer la consommation d'énergie.

Le procédé objet de l'invention consiste à effectuer périodiquement les opérations suivantes: on détermine pour chaque compartiment d'indice i la valeur optimale d'un paramètre choisi dans un premier groupe de cinq paramètres constitué par le débit d'alimentation en solution sucrée des compartiments rapporté au débit total de solution sucrée à traiter ($YEG_i$), le débit d'eau évaporée dans les compartiments rapporté au débit total d'eau évaporée dans l'appareil de cristallisation ($YEV_i$), le débit de masse cuite sortant des compartiments rapporté au débit total de masse cuite produite ($YMC_i$), le brix (teneur en matières sèches) de la masse cuite dans les compartiments ($BMC_i$) et la teneur en cristaux de sucre de la masse cuite dans les compartiments ($RDT_i$) en utilisant une loi préétablie donnant la valeur dudit paramètre dans lesdits compartiments en fonction du brix de la solution sucrée à traiter (BEGT), de la pression de vapeur dans la calandre de l'appareil de cristallisation (PCAL) et du rapport:

$$R = (QMAX \times RDTMAG)/(QEGT \times BEGT)$$

avec:
QMAG: débit de magma
RDTMAG: teneur en cristaux du magma
QEGT: débit total de solution sucrée à traiter
BEGT: brix de la solution sucrée à traiter

On calcule la valeur optimale d'un premier paramètre de conduite (PVAP ou QEGT) en utilisant pour ce calcul la valeur optimale déterminée pour ledit paramètre du premier groupe, les valeurs imposées d'un second paramètre de conduite (QEGT ou PVAP) et d'un paramètre d'un second groupe (paramètres d'état résultant) constitué par le débit total d'eau évaporée (QEV), le débit total de masse cuite produite (QMC), le brix de la masse cuite produite (BMC) et la teneur en cristaux de la masse cuite produite (RDT) et éventuellement un terme correctif dont la valeur est fonction de l'écart entre la valeur imposée dudit paramètre du second groupe et la valeur mesurée de celui-ci, et on règle ledit premier paramètre de conduite pour le maintenir à une valeur de consigne égale à la valeur optimale calculée pour ce paramètre.

Les paramètres de conduite sont des paramètres sur lesquels on peut agir directement par opposition aux paramètres d'état résultants dont les variations résultent d'une modification des paramètres de conduite.

Les lois donnant les variations des paramètres du premier groupe peuvent aussi être fonction du débit total de solution à traiter (QEGT) ou du débit total d'eau évaporée (QEV) ou du débit total de masse cuite (QMC) suivant le choix des paramètres à optimiser. Elles peuvent être présentées sous forme de relations mathématiques ou d'un fichier de courbes ou de tableaux.

Pour le calcul de la valeur optimale du paramètre de conduite, on pourra utiliser, à la place du paramètre du second groupe dont les valeurs imposées et mesurées doivent être comparées, une image physique ou chimique dudit paramètre; par exemple le brix de la masse cuite produite (BMC) pourra être remplacé par la densité ou la compacité ou la température de la masse cuite, la teneur en cristaux de sucre de la masse cuite produite (RDT) pourra être remplacée par la compacité de la masse cuite et le débit total d'eau évaporée (QEV) pourra être remplacé par le débit d'eau condensée dans le faisceau de chauffage de l'appareil de cristallisation. Il est bien entendu que dans la description et les revendications qui suivent toute désignation de l'un quelconque de ces paramètres comprendra le paramètre lui-même et ses grandeurs de remplacement.

Pour calculer la valeur optimale du premier paramètre de conduite on effectue un bilan massique global, un bilan massique matières sèches, un bilan thermique d'eau évaporée et un bilan de cristallisation. Ces calculs sont effectués à intervalles de temps réguliers, pouvant varier de 1 à 5 minutes, par un système de conduite automatisée comportant un ordinateur de supervision qui reçoit des informations, en provenance de différents appareils de mesure, sur certaines caractéristiques de la solution sucrée, du magma et de la masse cuite et sur les paramètres de marche de l'appareil de cristallisation ainsi que des données extérieures introduites par exemple au moyen d'un clavier, notamment les valeurs imposées de certains paramètres.

Des résultats particulièrement intéressants peuvent être obtenus en choisissant comme paramètre du premier groupe, dont la valeur optimale est déterminée à partir d'une relation mathématique ou d'un fichier de courbes ou de tableaux, le débit d'alimentation en solution sucrée des compartiments rapporté au débit total de solution sucrée à traiter ($YEG_i$), et comme premier paramètre de conduite, dont la valeur optimale est calculée, la pression de vapeur de chauffage (PVAP), les deux paramètres dont les valeurs sont imposées, en fonction de la gestion des jets de cristallisation en amont et en aval, étant le débit total de solution sucrée (QEGT) et le brix de la masse cuite produite (BMC). C'est ce mode de mise en œuvre qui est décrit ci-après, à titre d'exemple non limitatif, en se référant aux dessins l'accompagnant et sur lesquels:

La figure 1 est le schéma d'un système de conduite automatisée d'un appareil de cristallisation pour la mise en œuvre du procédé de l'invention,

La figure 2 est l'organigramme du procédé,

La figure 3 est une courbe de répartition du débit total de solution sucrée entre les compartiments, et

La figure 4 est une courbe des variations du débit de vapeur d'agitation en fonction du débit total de solution sucrée à traiter.

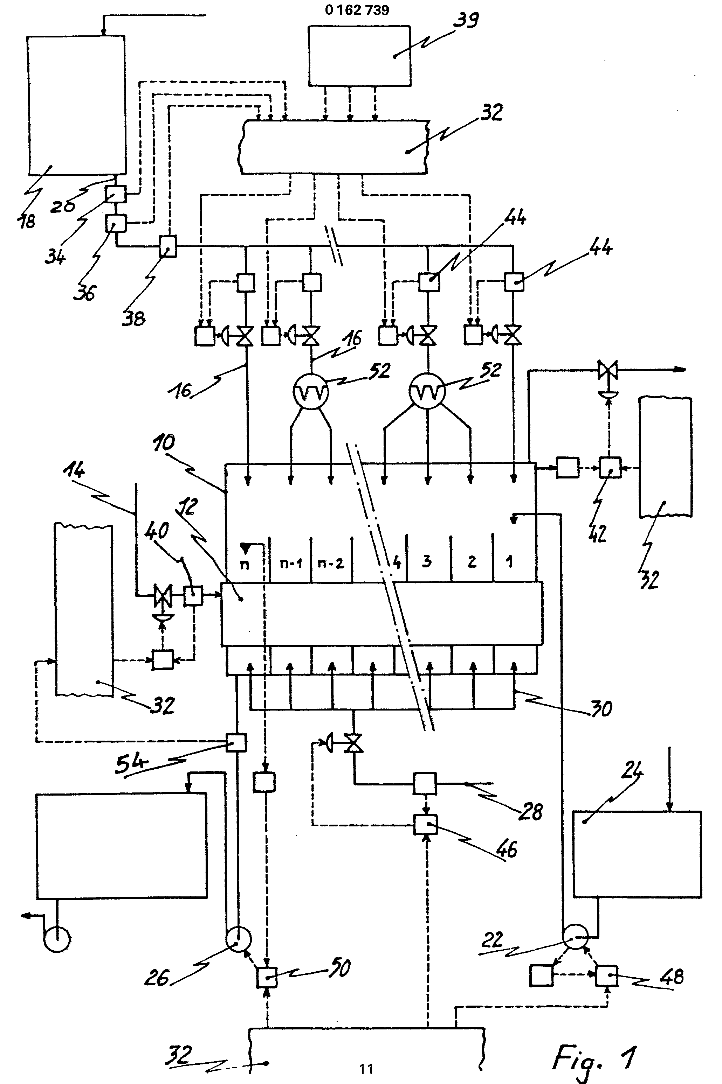

Sur la figure 1, le numéro de référence 10 désigne un appareil de cristallisation par évaporation à marche continue constitué par une cuve fermée disposée horizontalement et divisée par des cloisons verticales en plusieurs compartiments 1, 2, 3, 4, ..., n−2. n−1, n. Ces cloisons n'atteignent pas le sommet de la cuve de sorte que tous les compartiments communiquent entre eux à la partie supérieure de la cuve. Des ouvertures sont également prévues dans les cloisons pour permettre le passage de la masse cuite d'un compartiment à l'autre. Dans la partie inférieure de la cuve est placé un faisceau de chauffage 12 commun à tous les compartiments et formé de tubes ou de plaques dans lesquels on fait circuler de la vapeur amenée par une conduite 14.

Une solution sucrée, qui sera appelée égout dans ce qui suit, est distribuée dans les différents compartiments par des tuyauteries 16; cet égout provient d'un bac 18 auquel les tuyauteries 16 sont reliées par une conduite 20. On peut prévoir une tuyauterie d'alimentation par compartiment ou une tuyauterie commune à un groupe de compartiments.

Un magma constitué par un mélange de petits cristaux constituant des germes de cristallisation et de liqueur mère est introduit dans le premier compartiment au moyen d'une pompe à débit variable 22 prélevant le magma sur une capacité 24.

La masse cuite produite est extraite du dernier compartiment au moyen d'une pompe à débit variable 26.

De la vapeur est injectée à la partie inférieure de chaque compartiment, pour agiter la masse cuite et favoriser les échanges thermiques; cette vapeur amenée par une conduite 28 est distribuée à des injecteurs 30 placés dans le bas des compartiments.

Le numéro de référence 32 désigne un système de conduite automatisée se composant d'un ordinateur de supervision couplé à un système de conduite de processus comprenant les régulateurs numériques et l'instrumentation ou à des chaînes de régulation analogiques classiques. En variante, l'ordinateur peut assurer les fonctions de régulation et communiquer avec l'instrumentation par des convertisseurs digital-analogique et analogique-digital. Le système reçoit des informations numériques et analogiques, les traite et émet les consignes qu'il a optimisées vers les actionneurs; vannes, moteurs, etc. Pour simplifier la figure 1, les différents éléments constitutifs de ce système ont été répartis sur le dessin.

Le système 32 reçoit en permanence des informations sur la température, le débit total et le brix de l'égout qui sont mesurées par des instruments 34, 36 et 38, respectivement et des informations sur le brix de la masse cuite fournies par un instrument 54. Il reçoit en outre des données externes: résultats d'analyses de laboratoire, caractéristiques imposées par l'exploitant, etc., qui sont introduites par exemple au moyen d'un clavier 39. Il a également en mémoire les caractéristiques technologiques de l'appareil 10: dimensions et volume utile de la cuve, structure du faisceau de chauffage, dimension des tubes du faisceau, etc.

Le système comprend en outre un régulateur de la pression de la vapeur de chauffage 40, un régulateur 42 de la pression de vapeur dans la calandre de l'appareil, des régulateurs de débit partiel d'égout 44, un régulateur de débit de vapeur d'agitation 46, un régulateur de débit de magma 48 commandant la pompe 22 et un régulateur de niveau de masse cuite 50 commandant la pompe 26.

Conformément à la présente invention, on détermine à intervalles de temps réguliers les valeurs optimales des débits d'alimentation en égout de chaque compartiment ou groupe de compartiments, les débits étant rapportés au débit total de solution sucrée à traiter, du débit de magma admis dans le premier compartiment, de la pression de la vapeur de chauffage et du débit de vapeur d'agitation injecté dans les compartiments et on les utilise comme valeurs de consigne dans les régulateurs correspondants. Ces intervalles de temps pourront être compris, par exemple, entre une et cinq minutes. Les valeurs optimales des débits d'alimentation $YEG_i$ des compartiments sont déterminées au moyen d'un fichier donnant sous forme de courbes ou de tableaux la répartition en pourcentage du débit total QEGT entre les différents compartiments. Cette répartition dépend de différents paramètres, notamment de la pression de vapeur dans la calandre de l'appareil PCAL, du débit total d'égout à traiter QEGT, de son prix BEGT et du rapport:

$$R = (QMAG \times RDTMAG)/(QEGT \times BEGT)$$

QMAG étant le débit de magma introduit dans le premier compartiment et RDTMAG étant la teneur en cristaux du magma.

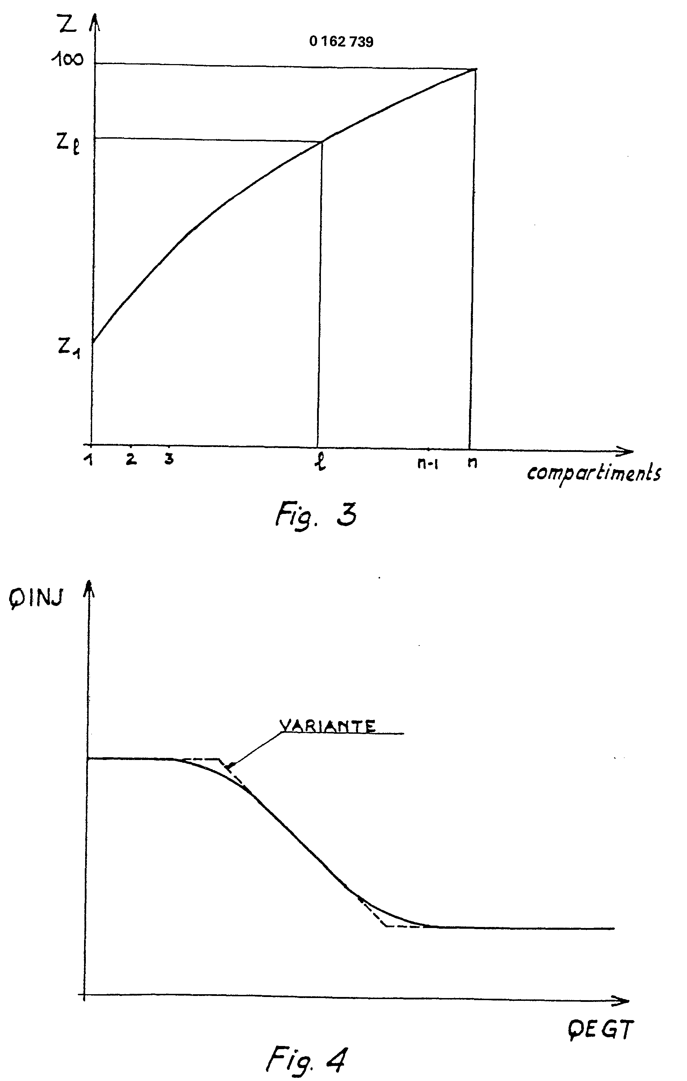

Le fichier contient plusieurs courbes ou tableaux correspondant à différentes valeurs de ces paramètres et le système de conduite automatisée sélectionne la courbe ou le tableau à utiliser en fonction des valeurs mesurées ou imposées de ces paramètres. La fig. 3 montre une telle courbe de répartition du débit total d'égout. On a porté en abscisse les numéros des compartiments et en ordonnées les débits cumulés en pourcentage du débit total.

La valeur optimale du débit de magma est calculée à l'aide de la formule:

$$R = (QMAG \times RDTMAG)/(QEGT \times BEGT)$$

à partir des valeurs mesurées de QEGT et BEGT, de la valeur de RDTMAG, déterminée à partir d'analyses en laboratoire du magma, et de la valeur de R fixée par l'exploitant.

La valeur optimale du débit total de vapeur d'agitation injecté dans les compartiments est déterminée au moyen d'un second fichier donnant sous forme de courbes ou de tableaux le débit de vapeur QINJ en fonction du débit total d'égout à traiter QEGT ou du débit de masse cuite produite QMC ou du débit total d'eau évaporée QEV. La relation entre QINJ et QEGT, QMC ou QEV, dépend de différents paramètres, notamment du brix de l'égout à traiter, de la pression de la calandre et du rapport R et ce fichier contient plusieurs courbes ou tableaux correspondant à différentes valeurs de ces paramètres. La figure 4 montre une courbe des variations du débit de vapeur d'agitation QINJ en fonction du débit d'égout QEGT. On pourrait aussi envisager de régler les débits de vapeur injectés compartiment par compartiment; dans ce cas, le fichier contiendrait autant de jeux de courbes ou de tableaux qu'il y a de compartiments.

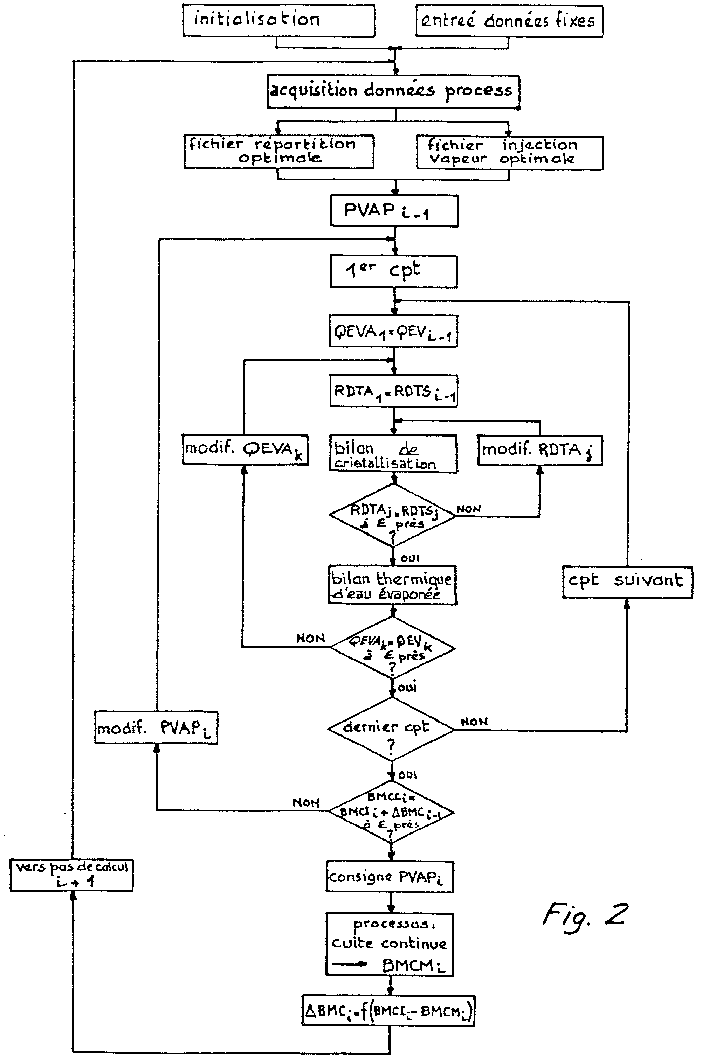

La valeur optimale de la pression de la vapeur de chauffage PVAP est calculée par le système 32 suivant un procédé schématisé sur la figure 2. Le système de conduite automatisée effectue pour chaque compartiment un bilan de cristallisation et un bilan thermique d'eau évaporée en utilisant les valeurs mesurées ou déterminées comme indiqué plus haut des différentes grandeurs caractérisant l'égout à traiter et le magma (débit, brix, température, etc.), ainsi que celles des différents paramètres caractérisant le fonctionnement de l'appareil (débits d'alimentation en égout, débit de vapeur d'agitation, pression de vapeur dans la calandre de l'appareil, niveau de masse cuite dans l'appareil, etc.). Pour effectuer le bilan de cristallisation le système 32 utilise les relations suivantes:

(1) $QMCX = QMCE + YEG_i \times QEGT - YEV_i \times QEV$

(2) $BMCS = (BMCE \times QMCE + BEGT \times YEG_i \times QEGT)/QMCS$

(3) $PMCS = (PMCE \times BMCE \times QMCE + PEGT \times BEGT \times YEG_i \times QEGT)/(QMCS \times BMCS)$

(4) $SD = K \times Sp \times M \times C \times (\delta - 1) \times \tau$

(5) $RDTS = \dfrac{SD + M}{QMCS}$

et

(6) $OMS = \left(\dfrac{QMCS \times RDTS}{QMCE \times RDTE}\right)^{\frac{1}{3}} \times OME$

QMCE, BMCE, PMCE, TMCE, RDTE et OME étant, respectivement le débit, le brix, la pureté, la température, la teneur en cristaux et la dimension moyenne des cristaux de la masse cuite entrant dans un compartiment, QMCS, BMCS, PMCS, TMCS, RDTS et OMS les grandeurs corespondantes pour la masse cuite sortant d'un compartiment, PEGT et TEGT la pureté et la température de l'égout, SD la masse de sucre déposée par cristallisation dans le compartiment, K le coefficient d'échange massique, Sp la surface spécifique des cristaux, M le débit massique des cristaux (M = QMCE × RDTE), C la concentration en sucre de l'eau mère dans le compartiment, δ la sursaturation de l'eau mère dans le compartiment, τ le temps de séjour dans le compartiment.

Pour les bilans thermiques d'eau évaporée, le système 32 utilise la formule suivante:

(7) $QEV_1 = (QMCE \times CpMCE \times TMCE + YEG_i \times QEGT \times CpEGT \times TEGT - QMCS \times CpMCS \times TMCS - \text{Pertes thermiques} + h \times \Delta S \times \Delta tu)/H$

CpMCE, CpMCS et CpEGT étant, respectivement, la chaleur spécifique de la masse cuite entrant dans le compartiment, de la masse cuite sortant du compartiment et de l'égout, h étant le coefficient d'échange thermique, ΔS la surface d'échange thermique du compartiment, Δtu l'écart de température utile, et H l'enthalpie de la vapeur de chauffage.

Pour le premier compartiment les valeurs de QMCE, BMCE, PMCE, RDTE, TMCE et CpMCE sont celles des grandeurs correspondantes du magma.

Pour chaque compartiment, le système 32 effectue d'abord un bilan de cristallisation pour calculer les valeurs des grandeurs caractéristiques de la masse cuite sortant du compartiment, notamment de sa teneur en cristaux RDTS, en fonction des valeurs des grandeurs caractéristiques de la masse cuite et de l'égout entrant dans le compartiment et des paramètres caractérisant la marche de l'appareil. Pour ce calcul, le système suppose connus la pression de la vapeur de chauffage PVAP, le débit d'eau évaporée dans le compartiment $YEV_i$ et la teneur en cristaux de la masse cuite sortant du compartiment RDTS. Au début de chaque pas de calcul d'indice i, le système attribue à ces grandeurs les valeurs $PVAP_{i-1}$, $YEV_{i-1}$ et $RDTS_{i-1}$ déterminée au pas de calcul précédent d'indice i−1. Si la valeur calculée RDTSi est égale, à la précision recherchée près, à $RDTS_{i-1}$, le système retient cette valeur et passe à l'étape suivante du calcul. Si non, un processus itératif permet de calculer une valeur RDTSj, j étant l'indice de cette boucle d'itération, à partir d'une valeur RDTAj attribuée par le système et fonction des valeurs précédentes RDTSj−1 et RDTAj−1 suivant une loi prédéterminée. Le calcul sera recommencé jusqu'à ce que la valeur calculée RDTSj soit égale avec l'approximation désirée, à la valeur RDTAj attribuée par le système; la valeur retenue de RDTSi sera alors égale à RDTSj.

Le système effectue alors le bilan thermique d'eau évaporée du compartiment. Les calculs de bilan de cristallisation et de bilan thermique d'eau évaporée sont recommencés jusqu'à ce que la valeur calculée QEVk soit égale, avec l'approximation désirée, à la valeur QEVAk attribuée par le système.

Le système effectue ainsi un bilan de cristallisation et un bilan thermique d'eau évaporée pour chacun des compartiments, du premier au dernier, en utilisant comme valeurs des grandeurs caractéristiques de la masse cuite entrant dans un compartiment les valeurs calculées pour la masse cuite sortant du compartiment précédent.

Lorsque les bilans ont été bouclés pour tous les compartiments, on compare la valeur calculée de l'une des grandeurs caractéristiques de la masse cuite sortant du dernier compartiment, c'est-à-dire la masse cuite extraite de l'appareil de cristallisation, à la valeur imposée pour cette grandeur. Dans l'exemple décrit on a choisi le brix de la masse cuite. La valeur calculée du brix BMCC est en fait comparée à la valeur imposée BMCI augmentée d'un terme correctif $\Delta BMC$, qui sera défini ci-après et qui peut être positif, négatif ou nul. Si ces deux valeurs sont égales, à la précision recherchée près, on arrête le calcul et on retient pour valeur optimale de la pression de vapeur de chauffage la valeur $PVAP_{i-1}$ attribuée par le système au début du calcul. Si non, on remplace cette valeur par une nouvelle valeur calculée suivant une loi déterminée en fonction de l'écart entre BMCI + $\Delta BMC$ et BMCC, et on recommence tous les calculs en utilisant cette nouvelle valeur. Si le brix BMCC calculé à partir de cette nouvelle valeur ne satisfait pas l'égalité BMCC = BMCI + $\Delta BMC$ on recommence l'ensemble des calculs après avoir à nouveau modifié la valeur de PVAP. Lorsque cette égalité est vérifiée avec l'approximation désirée, les calculs sont arrêtés et la dernière valeur de PVAP retenue comme valeur optimale de la pression de la vapeur de chauffage.

La valeur calculée du brix de la masse cuite BMCC est alors comparée à la valeur BMCM mesurée au moyen d'un instrument 54. Si ces deux valeurs diffèrent, le système calcule le terme correctif $\Delta BMC$ qui est fonction de la différence BMJI − BMCM. Ce terme correctif est mis en mémoire pour être utilisé au pas de calcul suivant comme décrit ci-dessus.

Les débits d'alimentation en égout des compartiments ou groupes de compartiments sont maintenus égaux aux valeurs optimales déterminées comme expliqué plus haut par les régulateurs 44 commandant des vannes placées sur les tuyauteries d'alimentation, ces valeurs optimales étant utilisées comme valeurs de consigne des régulateurs. En variante, on pourrait utiliser des vannes tout ou rien commandées par le système de conduite automatisée, le contrôle des débits s'effectuant alors en boucle ouverte à partir de la mesure du débit en position vanne ouverte et en jouant sur les temps d'ouverture et de fermeture des vannes.

La pression de la vapeur de chauffage est maintenue égale à la valeur optimale calculée comme décrit ci-dessus et utilisée comme valeur de consigne du régulateur 40. La pression dans la calandre de l'appareil de cristallisation est maintenue par le régulateur 42 à une valeur constante fixée par l'exploitant.

Les débits de vapeur d'agitation et de magma sont maintenus à des valeurs de consigne égales aux valeurs optimales de ces grandeurs par les régulateurs 46 et 48 commandant, respectivement, une vanne placée sur la tuyauterie 28 et le moteur d'entraînement de la pompe 22.

Le niveau de masse cuite dans l'appareil est maintenu à une valeur de consigne par le régulateur 50. On pourra maintenir ce niveau constant ou, en variante, admettre des variations de niveau, sur une plage étroite, par exemple pour maintenir le malaxeur aval plein en permanence. De même, en cas de variation importante du débit aval, imposée par la cadence d'essorage par exemple, le débit de la pompe d'extraction de masse cuite sera modifié pour maintenir le niveau du malaxeur aval et les débits d'égout et de magma seront modifiés en conséquence, dans des limites imposées en valeur haute par la pression maximum de vapeur de chauffage et en valeur basse par le niveau haut des capacités tampons placées en amont du cristalliseur. En cas de dépassement de ces limites, une limitation des débits des ateliers amont ou aval, suivant le cas, sera nécessaire.

Les fichiers utilisés pour déterminer les valeurs optimales des débits d'alimentation des compartiments et du débit de la vapeur d'agitation, pourraient être remplacés par des relations mathématiques traduisant les lois:

$$YEG_i = f(QEGT, BEGT, R, PCAL) \text{ et}$$
$$QINJ = h(QEGT, BEGT, R, PCAL)$$

respectivement.

Les grandeurs et paramètres non imposés par l'exploitant qui interviennent dans le procédé de conduite automatisée objet de la présente invention peuvent être soit mesurés en continu, soit déterminés et introduits dans le système périodiquement, par exemple au moyen d'un clavier.

Les calculs permettant de déterminer la valeur optimale de PVAP donnent également les valeurs optimales de QEV, QMC et RDT et celles-ci peuvent être comparées aux valeurs mesurées de ces grandeurs pour déceler éventuellement une dérive du système de conduite et la corriger.

Comme cela résulte de la définition générale de l'invention donnée dans le préambule, de nombreuses autres formes de mise en œuvre sont possibles en choisissant différemment le paramètre dont la valeur optimale est déterminée au moyen d'un fichier ou d'une relation mathématique et les deux paramètres dont les valeurs sont imposées. Ce choix dépendra du but visé: production maximale de sucre, réduction de la consommation d'énergie, etc.

## Revendications

1. Procédé de conduite automatisée d'un appareil de cristallisation par évaporation à marche continue pour la production de sucre, constitué par une cuve fermée disposée horizontalement et divisée par des cloisons verticales en plusieurs compartiments munis d'un faisceau de chauffage, une solution sucrée étant introduite dans chacun des compartiments, un magma formé d'un mélange de petits cristaux et de liqueur mère étant introduit dans le premier compartiment et la masse cuite produite étant extraite du dernier compartiment, caractérisé en ce qu'on effectue périodiquement les opérations suivantes: on détermine, pour chaque compartiment d'indice i la valeur optimale d'un paramètre choisi dans un premier groupe de cinq paramètres constitué par le débit d'alimentation en solution sucrée des compartiments rapporté au débit total de solution sucrée à traiter ($YEG_i$), le débit d'eau évaporée dans les compartiments rapporté au débit total d'eau évaporée dans l'appareil de cristallisation ($YEV_i$), le débit de masse cuite sortant des compartiments rapporté au débit total de masse cuite produite ($YMC_i$), le brix de la masse cuite dans les compartiments ($BMC_i$) et la teneur en cristaux de sucre de la masse cuite dans les compartiments ($RDT_i$), en utilisant une loi préétablie donnant la valeur dudit paramètre dans les compartiments en fonction du brix de la solution sucrée à traiter (BEGT), de la pression de vapeur dans la calandre de l'appareil de cristallisation (PCAL) et du rapport:

$$R = (QMAG \times RDTMAG)/(QEGT \times BEGT)$$

avec:
QMAG: débit de magma
RDTMAG: teneur en cristaux du magma
QEGT: débit total de solution sucrée à traiter
BEGT: brix de la solution sucrée à traiter
on calcule la valeur optimale d'un premier paramètre de conduite (PVAP — pression de vapeur de chauffage — ou QEGT) en utilisant pour ce calcul la vapeur optimale déterminée pour ledit paramètre du premier groupe, les valeurs imposées d'un second paramètre de conduite (QEGT ou PVAP) et d'un paramètre d'un second groupe (paramètres d'état résultant) constitué par le débit total d'eau évaporée (QEV), le débit total de masse cuite produite (QMC), le brix de la masse cuite produite (BMC) et la teneur en cristaux de la masse cuite produite (RDT) et éventuellement un terme correctif dont la valeur est fonction de l'écart entre la valeur imposée dudit paramètre du second groupe et la valeur mesurée de celui-ci, et on règle ledit premier paramètre de conduite pour le maintenir à une valeur de consigne égale à la valeur optimale calculée pour ce paramètre.

2. Procédé selon la revendication 1, caractérisé en ce que les lois donnant les variations des paramètres du premier groupe sont fonction du débit total de solution à traiter (QEGT) ou du débit total d'eau évaporée (QEV) ou du débit total de masse cuite produite (QMC), suivant le choix des paramètres du second groupe à optimiser.

3. Procédé selon la revendication 1 ou 2, caractérisé en ce que la valeur optimale pour chaque compartiment du paramètre du premier groupe est déterminée au moyen d'une relation mathématique ou d'un fichier de courbes ou de tableaux donnant les valeurs de ce paramètre pour les différents compartiments pour différentes valeurs du brix de la solution sucrée à traiter (BEGT), dudit rapport (R), de la pression de vapeur dans la calandre de l'appareil de cristallisation (PCAL) et éventuellement du débit total de la solution sucrée à traiter (QEGT) ou du débit total de masse cuite produite (QMC) ou du débit total d'eau évaporée (QEV).

4. Procédé selon la revendication 1 ou 2, caractérisé en ce qu'on utilise, à la place du paramètre du second groupe dont on compare les valeurs imposées et mesurées, une grandeur représentative dudit paramètre.

5. Procédé selon la revendication 1, 2, 3 ou 4, caractérisé en ce que, pour calculer la valeur optimale du premier paramètre de conduite, on effectue les bilans de cristallisation et thermique d'eau évaporée pour chaque compartiment.

6. Procédé selon l'une quelconque des revendications précédentes, caractérisé en ce qu'on détermine périodiquement les valeurs optimales des débits d'alimentation en solution sucrée de chaque compartiment en utilisant une loi de répartition du débit dans les compartiments qui est fonction du débit total de solution à traiter (QEGT), du brix de la solution (BEGT), dudit rapport (R) et de la pression de vapeur dans la calandre de l'appareil (PCAL), on calcule la valeur optimale de la pression de vapeur de chauffage (PVAP) en utilisant comme paramètres imposés le débit total de solution sucrée (QEGT), le brix de la masse cuite produite (BMC) et éventuellement un terme correctif (ΔBMC) dont la valeur est fonction de l'écart entre la valeur imposée du brix de la masse cuite (BMCI) et une valeur mesurée de ce brix (BMCM), et on règle la pression de vapeur de chauffage pour la maintenir à une valeur de consigne égale à la valeur optimale calculée.

7. Procédé selon la revendication 6, caractérisé en ce que la pression de vapeur dans la calandre de l'appareil de cristallisation (PCAL) est maintenue constante.

8. Procédé selon la revendication 6 ou 7, caractérisé en ce que le débit de magma introduit dans le premier compartiment (QMAG) est réglé de façon à maintenir ledit rapport (R) constant.

9. Procédé selon la revendication 6, 7 ou 8, caractérisé en ce qu'on injecte de la vapeur dans chaque compartiment, à sa partie inférieure, on détermine la valeur optimale du débit de vapeur injectée (QINJ) au moyen d'une ou plusieurs relations mathématiques ou d'un jeu de courbes ou de tableaux donnant les variations de ce débit en fonction du débit total de solution sucrée à traiter (QEGT) pour différentes valeurs du brix de la solution (BEGT), dudit rapport (R) et de la pression de vapeur dans la calandre de l'appareil de cristallisation (PCAL) et on règle le débit de vapeur injectée pour le maintenir à une valeur de consigne égale à cette valeur optimale.

**Patentansprüche**

1. Verfahren zur automatischen Bedienung eines durchlaufenden Verdampfungskristallisationsapparates für die Zuckerherstellung, bestehend aus einem horizontal liegenden geschlossenen Behälter, das durch senkrechte Wände in mehrere, mit einer Heizkammer versehene Abteile geteilt ist, wobei eine Zuckerlösung in jedes Abteil eingeführt wird, ein aus einem Gemisch von kleinen Kristallen und Mutterlösung gebildetes Magma in das erste Abteil eingeführt wird und die erzeugte Kochmasse aus dem letzten Abteil ausgezogen wird, dadurch gekennzeichnet, dass man folgende Vorgänge periodisch vornimmt: man bestimmt, für jedes Abteil mit Indiz i, den optimalen Wert eines in einer ersten Gruppe von fünf Parametern ausgewählten Parameters, wobei diese Gruppe aus der in die Abteile eingespeisten Zuckerlösungsmenge bezogen auf die Gesamtmenge der zu verarbeitenden Zuckerlösung (YEGi), der Menge vom in den Abteilen verdampften Wasser bezogen auf die Gesamtmenge vom im Kristallisationsapparat verdampften Wasser (YEVi), der Menge der aus den Abteilen ausgezogenen Kochmasse bezogen auf die Gesamtmenge der erzeugten Kochmasse (YMCi), dem Brix-Grad der Kochmasse in den Abteilen (BMCi) und dem Zuckergehalt der Kochmasse in den Abteilen besteht, indem man ein vorherbestimmtes Gesetz anwendet, das den Wert des obenerwähnten Parameters in den Abteilen in Abhängigkeit vom Brix-Grad der zu verarbeitenden Zuckerlösung (BEGT), vom Dampfdruck im Brüdenraum des Kristallisationsapparates (PCAL) und vom Verhältnis

$$R = (QMAG \times RDTMAG)/(QEGT \times BEGT)$$

wobei
QMAG: Magmamenge
RDTMAG: Magmakristallgehalt
QEGT: Gesamtmenge der zu verarbeitenden Zukerlösung
BEGT: Brix-Grad der zu verarbeitenden Zuckerlösung
ergibt, man den Optimalen Wert eines ersten Bedienungsparameters (PVAP – Heizdampfdruck – oder QEGT) berechnet, indem man für diese Berechnung den für den Obenerwähnten Parameter der ersten Gruppe ermittelten optimalen Wert, die vorgegebenen Werte eines zweiten Bedienungsparameters (QEGT oder PVAP) und eines Parameters einer zweiten Gruppe (resultierendes Zustandsparameter) anwendet, die aus der Gesamtmenge vom verdampften Wasser (QEV), der Gesamtmenge der erzeugten Kochmasse (QMC), dem Brix-Grad der erzeugten Kochmasse (BMC) und dem Kristallgehalt der erzeugten Kochmasse (RDT) sowie eventuell aus einem Korrekturglied besteht, dessen Wert von der Differenz zwischen dem vorgegebenen Wert des Obenerwähnten Parameters der zweiten Gruppe und dessen gemessenem Wert abhängt, und man den ersten Bedienungsparameter so regelt, dass er bei einem Sollwert erhalten bleibt, der dem für diesen Parameter berechneten optimalen Wert gleich ist.

2. Verfahren nach Anspruch 1, dadurch gekennzeichnet, dass die Gesetze, die die Änderungen der Parameter der ersten Gruppe ergeben, von der Gesamtmenge der zu verarbeitenden Lösung (QEGT) oder von der Gesamtmenge vom verdampften Wasser (QEV) oder von der Gesamtmenge der erzeugten Kochmasse (QMC) abhängen, je nach der Wahl der zu optimierenden Parameter der zweiten Gruppe.

3. Verfahren nach Anspruch 1 oder 2, dadurch gekennzeichnet, dass der für jedes Abteil optimale Wert des Parameters der ersten Gruppe mit Hilfe von einer mathematischen Relation, einer Kurven- oder Tabellendatei ermittelt wird, die die Werte dieses Parameters in den verschiedenen Abteilen für verschiedene Werte des Brix-Grades der zu verarbeitenden Zuckerlösung (BEGT), des Verhältnisses (R), des Dampfdruckes im Brüdenraum des Kristallisationsapparates (PCAL) und eventuell der Gesamtmenge der zu verarbeitenden Zuckerlösung (QEGT) oder der Gesamtmenge der erzeugten Kochmasse (QMC) oder der Gesamtmenge vom verdampften Wasser (QEV) geben.

4. Verfahren nach Anspruch 1 oder 2, dadurch gekennzeichnet, dass man anstatt des Parameters der zweiten Gruppe, von dem man die vorgegebenen mit den gemessenen Werten vergleicht, eine für diesen Parameter repräsentative Grösse anwendet.

5. Verfahren nach Anspruch 1, 2, 3 oder 4, dadurch gekennzeichnet, dass man zur Berechnung des optimalen Wertes des ersten Bedienungsparameters die Kristallisationsbilanz und die Wärmebilanz vom verdampften Wasser für jedes Abteil durchführt.

6. Verfahren nach irgendeinem der vorhergehenden Ansprüche, dadurch gekennzeichnet, dass man die optimalen Werte der in jedes Abteil eingespeisten Zuckerlösungsmenge periodisch ermittelt, indem man ein Gesetz der Mengenverteilung in den Abteilen anwendet, das von der Gesamtmenge der zu verarbeitenden Lösung (QEGT), dem Brix-Grad der Lösung (BEGT), des Verhältnisses (R) und dem Dampfdruck im Brüdenraum des Apparates (PCAL) abhängt, man den optimalen Wert des Heizdampfdrucks (PVAP) berechnet, in dem man als vorgegebene Parameter die Zuckerlösungsgesamtmenge (QEGT), den Brix-Grad der erzeugten Kochmasse (BMC) und eventuell ein Korrekturglied (ΔBMC) anwendet, dessen Wert von der Differenz zwischen dem vorgegebenen Wert des Brix-Grades der Kochmasse (BMCI) und einem gemessenen Wert dieses Brix-Grades (BMCM) abhängt, und man den Heizdampfdruck so regelt, dass er bei einem Sollwert erhalten bleibt, der dem berechneten optimalen Wert gleich ist.

7. Verfahren nach Anspruch 6, dadurch gekennzeichnet, dass der Dampfdruck im Brüdenraum des Kristallisationsapparates (PCAL) konstant gehalten wird.

8. Verfahren nach Anspruch 6 oder 7, dadurch gekennzeichnet, dass die Menge des in das erste Abteil eingeführten Magmas (QMAG) so geregelt ist, dass das Verhältnis (R) konstant erhalten bleibt.

9. Verfahren nach Anspruch 6, 7 oder 8, dadurch gekennzeichnet, dass man in jedes Abteil am dessen Unterteil Dampf injiziert, man den optimalen Wert der Menge vom injizierten Dampf (QINJ) mit Hilfe von einer oder mehreren mathematischen Relationen oder einem Satz Kurven oder von Tabellen ermittelt, die die Änderung dieser Menge in Abhängigkeit von der Gesamtmenge der zu verarbeitenden Zuckerlösung (QEGT) für verschiedene Werte des Brix-Grades der Lösung (BEGT), des Verhältnisses (R) und des Dampfdrucks im Brüdenraum des Kristallisationsapparates (PCAL) geben, und man die Menge vom injizierten Dampf so regelt, dass sie bei einem Sollwert erhalten bleibt, der diesem optimalen Wert gleich ist.

**Claims**

1. Process for automatically operating a continuously operating apparatus for crystallization by evaporation for producing sugar consisting of a closed horizontal vessel divided by vertical partitions into several compartments fitted with a heating bundle, a sugar solution being introduced into each compartment, a magma made up of a mixture of small crystals and mother liquor being introduced into the first compartment and the massecuite produced being withdrawn from the last compartment, characterized by the fact that the following operations are periodically carried out: for each "i" index compartment the optimal value of a parameter selected from a first group of five parameters constituted by the ratio of the sugar solution flow-rate fed to the compartments to the total flow-rate of sugar solution to the processed (YEGi), the ratio of the flow-rate of the water evaporated in the compartments to the total flow-rate of the water evaporated in the crystallization apparatus (YEVi), the ratio of the flow-rate of the massecuite coming from the compartments to the total flow-rate of the massecuite procuded (YMCi), the brix of the massecuite in the compartments (BMCi) and the sugar crystal content of the massecuite in the compartments (RDTi) is determined, using a pre-established law giving the value of the said parameter in the compartments according to the brix of the sugar solution to be processed (BEGT), the steam pressure in the vapor space of the crystallization apparatus (PCAL) and the ratio:

$$R = (QMAG \times RDTMAG)/(QEGT \times BEGT)$$

where
QMAG: magma flow-rate
RDTMAG: crystal content in magma
QEGT: total flow-rate of the sugar solution to be processed
BEGT: brix of the sugar solution to be processed, the optimal value of a first operation parameter (PVAP—heating steam pressure—or QEGT) is calculated using for this calculation the optimal value determined for the said parameter of the first group, the imposed values of a second operation parameter (QEGT or PVAP) and of a parameter of a second group (resulting status parameter) constituted by the total flow-rate of the evaporated water (QEV), the total flow-rate of the massecuite produced (QMC), the brix of the massecuite produced (BMC) and the crystal content of the massecuite produced (RDT) and, possibly, a corrective factor the value of which depends on the difference between the imposed value of the said parameter of the second group and the measured value of the latter, and the said first operation parameter is adjusted to maintain the same at a set value equal to the optimal value calculated for this parameter.

2. Process according to claim 1, characterized by the fact that the laws governing the fluctuations of the first group parameters depend on the total flow-rate of the solution to be processed (QEGT) or on the total flow-rate of the evaporated water (QEV) or on the total flow-rate of the massecuite produced (QMC), depending on the selection of the second group parameters to be optimized.

3. Process according to claim 1 or 2, characterized by the fact that the optimal value, for each compartment, of the first group parameter is determined, using a mathematical relationship or a file of curves or tables showing the values of this parameter for the various compartments for the various values of the brix of the sugar solution to be processed (BEGT), of the said ratio (R), of the steam pressure in the vapor space of the crystallization apparatus (PCAL) and, possibly, of the total flow-rate of the sugar solution to be processed (QEGT) or of the total flow-rate of the massecuite produced (QMC) or of the total flow-rate of the evaporated water (QEV).

4. Process according to claim 1 or 2, characterized by the fact that, instead of the second group parameter of which the imposed and measured values are compared, a representative figure of the said parameter is used.

5. Process according to claim 1, 2, 3 or 4, characterized by the fact that, in order to calculate the optimal value of the first operation parameter, a crystallization balance and an evaporated water thermal balance for each compartment are made.

6. Process according to any of the preceding claims, characterized by the fact that the optimal values of the sugar solution flow-rates fed to each compartment are periodically determined using a law of flow-rate distribution in the compartments which is a function of the total flow-rate of the solution to be processed (QEGT), of the brix of the solution (BEGT), of the said ratio (R) and of the steam pressure in the vapor space of the apparatus (PCAL), the optimal value of the heating steam pressure (PVAP) is calculated using as imposed parameters the total flow-rate of the sugar solution (QEGT), the brix of the massecuite produced (BMC) and, possibly, a corrective factor (ΔBMC) the value of which is a function of the difference between the imposed value of the brix of the massecuite (BMCI) and a measured value of this brix (BMCM), and the heating steam pressure is adjusted to maintain it at a set value equal to the optimal value calculated.

7. Process according to claim 6, characterized by the fact that the steam pressure in the vapor space of the crystallization apparatus (PCAL) is kept constant.

8. Process according to claim 6 or 7, characterized by the fact that the flow-rate of the magma introduced into the first compartment (QMAG) is adjusted so as to keep the said ratio (R) constant.

9. Process according to claim 6, 7 or 8, characterized by the fact that steam is injected into each compartment, at each bottom, by the fact that the optimal value of the flow-rate of the steam injected (QINJ) is determined using one or several mathematical relationships or a set of curves or tables giving the variations of this flow-rate according to the total flow-rate of the sugar solution to be processed (QEGT) for various values of the brix of the solution (BEGT), of the ratio (R) and of the steam pressure in the vapor space of the crystallization apparatus (PCAL), and the flow-rate of the steam injected is adjusted to keep it at a set value equal to this optimal value.

0 1 6 2 7 3 9

*Fig. 1*

0 162 739

initialisation

entreé données fixes

acquisition données process

fichier répartition optimale

fichier injection vapeur optimale

$PVAP_{i-1}$

$1^{er}$ cpt

$QEVA_1 = QEV_{i-1}$

$RDTA_1 = RDTS_{i-1}$

modif. $QEVA_k$

bilan de cristallisation

modif. $RDTA_j$

$RDTA_j = RDTS_j$ à $\varepsilon$ près ? — NON

OUI

bilan thermique d'eau évaporée

$QEVA_k = QEV_k$ à $\varepsilon$ près ? — NON

oui

cpt suivant

dernier cpt ? — NON

modif. $PVAP_L$

oui

$BMCC_i = BMCI_i + \Delta BMC_{i-1}$ à $\varepsilon$ près ? — NON

vers pas de calcul $i+1$

consigne $PVAP_i$

processus: cuite continue $\longrightarrow BMCM_i$

$\Delta BMC_i = f(BMCI_i - BMCM_i)$

Fig. 2

13

0 162 739

Fig. 3

Fig. 4

15